# EUROPEAN PATENT APPLICATION

(11) **EP 3 028 728 A1**
(43) Date of publication of application: **08.06.2016**
(21) Application number: 13890279.6
(22) Date of filing: 29.07.2013
(51) Int. Cl.: A61M 5/158

(54) **NEEDLE COVER AND WINGED NEEDLE WITH NEEDLE COVER**

(71) Applicant: Kawasumi Laboratories, Inc., Oita 876-0121 (JP)
(72) Inventor: WATANABE, Masatoshi, Bungo-Ono-shi Oita 8760121 (JP)
(74) Representative: Hering, Hartmut
(86) International application number: PCT/JP2013/070439
(87) International publication number: WO 2015/015547

(57) **Abstract**

Provided is a needle cover with good manipulability wherein, when a winged needle is drawn into the covert main body, the cover main body is easily expanded, rattling of the wings is minimized, allowing stably fixing the wings.

The cover main body (2) has a needle introduction part (3) and a needle introduction part (5). An opening (30P) is formed on the bottom (D) side of the needle introduction part (3), and the needle protection part (5) has a first needle protection part (the top protection part)(5.1) on the top (U) side and a second needle protection part (the bottom protection part) (5.2) on the bottom (D) side. An opening (5OP) is formed between the bottom (D) side of the first needle protection part (5.1) and the top side of the second needle protection part (5.2), and the first needle protection part (5.1) and the second needle protection part (5.2) are connected on the base (PE) side by a needle protection part connection unit (5H) and have a terminal opening (5OPDE) on the terminal (DE) side. On the base (PE) side, a wing-fixing unit (7) is formed on the first side (S1) and the second (S2) of the second needle protection part (5.2), and an locking part (7T) of the wing-fixing unit (7) is formed on the terminal (DE) side.

## Description

### TECHNICAL FIELD

The present invention relates to a needle cover of a winged needle of, for example, a PSV (pediatric scalp vein) set and an AVF (arteriovenous fistula) set, used for dialysis, transfusion, and blood transfusion, etc. The present invention more particularly relates to an improvement in a winged needle with a needle cover and relates to the invention significantly increasing manipulability while maintaining the reliability and safety of the needle.

### BACKGROUND ART

As conventional needle cover of a winged needle, for example, such inventions as described in Patent Documents 1 to 3 have been proposed.

The needle cover described in patent document 1 has a cover main body and the cover main body has a needle protection portion (wall) and two types of openings.

The needle protection portion (wall) has walls on a top U side and both side-portions (a first side-portion S1 and a second side-portion S2).

The two types of the openings are a narrow first opening and a second opening (formed larger than the first opening).

The first opening is formed along a longitudinal L direction of the walls on the both sides of the needle and the second opening is formed continuously to the first opening.

The needle is removed from a patient by pulling a needle hub or a tube connected thereto and thus drawing the needle inside the cover main body.

In this case, wings move from a terminal DE side to a base PE side along the narrow first opening and are fixed at an edge of the second opening.

The needle cover described in Patent Document 2 is an improvement of the invention described in Patent Document 1.

In particular, the terminal DE side of the narrow first opening (an entrance of a wing part) is formed larger. This facilitates the insertion of the wing part.

In addition, the narrow first opening is inclined upward to facilitate the insertion of the wing part and the second opening is inclined downward to prevent the fixed wings from moving in the opposite direction.

Further, a handle is formed on the terminal DE side of the cover main body. This enables an operator to hold firmly the handle to prevent the cover main body from moving backward together with the needle, thereby facilitating the winged needle to be easily pulled into the cover main body.

The needle cover described in Patent Document 3 is a still further improvement of the invention described in Patent Document 2. In particular, a third opening is provided between the narrow first opening and the second opening.

Thus constructed, in the needle cover of Document 3, a side wall of the cover main body is given flexibility, so that wings can be held more stably. This can also effectively prevent the wings from being accidentally detached from the cover main body, or can prevent a needle tip from accidentally protruding outwardly in a side S direction, after passing through the narrow first opening.

### PRIOR ART DOCUMENT

### PATENT DOCUMENTS

Patent Document 1: Japanese Examined Patent Application Publication No. 4-36026 (claims, Figs. 2, 3, and 5) (corresponding US patents: US5112311, US5266072, and US5433703)
Patent Document 2: Japanese Patent No. 3702408 (claims, Figs. 2, 5 to 12, and 14) (corresponding US patents: US5562636, US5562637, US5951529, and US6595965)
Patent Document 3: Japanese Patent No. 3809563 (claims, Figs. 1, 2, 5, 6, and 7 to 9) (corresponding US patents: US5704924 and US5772638)

### SUMMARY OF THE INVENTION

### PROBLEMS TO BE SOLVED BY THE INVENTION

As described above, the inventions of Patent Documents 1 to 3 include the narrow first opening facilitating the movement of the wings and the second opening fixing the wings, both formed in the needle protection portion (walls on the both side-portions) and therefore have the following drawbacks, in particular, regarding the difficulties in operation or in use as follows:
<A> since the region of second opening fixing the wings is limited to a partial portion on the terminal DE side of the cover main body, the fixation position can only be secured or allowed in the very vicinity of the needle;
<B> a thin side wall of the cover main body makes it difficult to firmly fix the wings;
<C> a method of fixing the wings is limited;
<D> an opening area of the first opening on the terminal DE side is limited to the originally prescribed size; and
<E> the widths of the first opening and the second opening are fixed and have substantially no room for change.

### MEANS FOR SOLVING PROBLEMS

As a result of intensive studies for solving the problems, the present inventor conceived the following invention:
[1] The present invention relates to and provides a needle cover (1, 1', 1") housing and protecting a winged needle after its use, the needle cover (1, 1', 1") having: a main body 2 and having a terminal part 2DE, an approximately middle part 2M, and a base part 2PE in a longitudinal L direction of the main body 2,
   the main body 2 having a needle introduction part 3 on the terminal part 2DE side, the needle introduction part 3 having an opening 30P formed on a bottom D side, the main body 2 having a base-side opening 20P on the base part 2PE side,
   the main body 2 having a needle protection part 5 over the approximately middle part 2M and the base part 2PE, the needle protection part 5 having a first needle protection part 5.1 on an top U side and having a second needle protection part 5.2 on a bottom D side,
   the needle cover (1, 1', 1") having an opening 50P formed between the bottom D side of the first needle protection part 5.1 and the top U side of the second needle protection part 5.2,
   the first needle protection part 5.1 and the second needle protection part 5.2 being connected on the base PE side by a needle protection part connection unit 5H and having a terminal opening 50PDE on the terminal DE side,
   the second needle protection part 5.2 having a wing-fixing unit 7 formed on the base PE side and also a first side S1 and a second side S2,
   the wing-fixing unit 7 having a locking part 7T formed on the terminal DE side.
[2] The present invention provides the needle cover (1, 1', 1") according to [1], wherein the needle cover (1, 1', 1") has an opening 70P formed between the top U side of the wing-fixing unit 7 and the bottom D side of the first needle protection part 5.1, and wherein the opening 70P is formed continuously to the base PE side of the opening 50P.
[3] The present invention provides the needle cover (1, 1', 1") according to [1] or [2], wherein the opening 5OP has the width or gap size made substantially the same from the terminal DE side to the base PE side.
[4] The present invention provides the needle cover (1, 1') according to any one of [1] to [3], wherein
   the first needle protection part 5.1 has a pair of legs 8 separated by a gap 80P and formed on the bottom D side in the longitudinal L direction as well as the first side S1 and second side S2 directions of the needle cover 1 from the terminal DE side to the base part 2PE, wherein
   the legs 8 are connected to the wing-fixing unit connection unit 7H on the base PE side as well as the bottom D side of the base part 2PE of the main body 2, and wherein
   the legs 8 are connected to a base PE side end of the needle introduction part 3 at a terminal DE side end of the main body 2.
[5] The present invention provides the needle cover (1) according to any one of [1] to [4], wherein
   a gap 5.1OP is formed between the terminal DE side of the first needle protection part 5.1 and the base PE side of the needle introduction part 3.
[6] The present invention provides the needle cover (1') according to any one of [1] to [4], wherein
   a gap 5.1OP' is formed in the approximately middle part of the first needle protection part 5.1, and wherein
   a terminal side first needle protection part 5.11 and a base side first needle protection part 5.12 are formed.
[7] The present invention provides the needle cover (1") according to any one of [1] to [4], wherein
   at least on the terminal DE side, the second needle protection part 5.2 has a width W2 in the side S direction made smaller than a width W1 in the side S direction of the first needle protection part 5.1, wherein
   a pair of engaging parts 9 is formed on the first needle protection part 5.1 and the second needle protection part 5.2, and wherein
   the engaging parts 9 has a first engaging part 9.1 formed on the first needle protection part 5.1 and a second engaging part 9.2 formed on the second needle protection part 5.2.
[8] The present invention provides the needle cover (1, 1', 1") according to any one of [1] to [7], wherein
   a handle 6 is formed at a terminal DE side end of the needle introduction part 3, and wherein the handle 6 is formed to erect from a horizontal axis HL toward the top U side and formed in an approximately plate shape or an approximately band shape.
[9] The present invention provides the needle cover (1', 1") according to any one of [1] to [4] and [6] to [8], wherein
   the needle introduction part 3 has a bottom wall 3WU' formed to incline upward in the terminal DE direction relative to the horizontal axis HL to have an inclination angle θ.
[10] The present invention provides a winged needle with a needle cover comprising: a winged needle 30; and the needle cover (1, 1', 1") according to any one of [1] to [9].

### ADVANTAGEOUS EFFECTS OF THE INVENTION

The present invention has the following advantageous effects:
[1] Since the needle protection part 5 (the first needle protection part 5.1 and the second needle protection part 5.2) and the wing-fixing unit 7 are separately formed,
   <1> the wings 35 can be fixed to the wing-fixing unit 7 at a position far from the needle 31. Therefore, the firm and stable fixation of the wings 35 can be achieved.
   <2> Since the wings 35 can extensively be fixed over a wide ranged-area of the wing-fixing unit 7, the wings 35 can be firmly and stably be secured.
[2] Having equipped with above mentioned [1], the needle protection part 5 (the first needle protection part 5.1 and the second needle protection part 5.2) (a so-called accidental puncture prevention portion) can be so formed as to have a minimum inner diameter, that means the minimum diameter allowing a tube T just to pass through, which makes it possible to reduce the gap between the outer diameter of the tube T and the outer diameter of the needle 31 to the minimum. Thus constituted, the winged needle 31 can be drawn into the cover main body 2 with just minimized rattling and with enhanced stable manipulability.
[3] Having equipped with above [1], the opening 50P allowing passage of the wings 35, can be formed in a straight shape (approximately linear shape), which enables the wings 35 to be inserted into the opening 50P without much resistance thereby provided with improved manipulability.
[4] The needle protection part 5 is divided into two parts, i.e., the first needle protection part 5.1 and the second needle protection part 5.2., which forms an opening 5OP therebetween. Thus constituted, when the winged needle 30 is drawn into the cover main body 2, an opening area of the opening 50PDE of the opening 50P at the terminal DE side can be expanded widely to facilitate the insertion of the wings 35.
[5] In addition to above [4], since the handle 6 is formed on the portion of the terminal DE side of the first needle protection part 5.1 and the position of the handle 6 can be varied up and down when an operator pulls the handle 6 with fingers, the needle cover can be used regardless of the size of the hands of the operator.
[6] In addition to above [4], when the handle 6 is lifted, the opening 70P of the wing-fixing unit 7 is also widened at the same time, thereby allowing the wings 35 to be inserted into the wing-fixing unit 7 without much resistance.
[7] Having equipped with above [4], (as in the third embodiment) the width in the side S direction of the second needle protection part 5.2 can be made smaller than the width in the side S direction of the first needle protection part 5.1 so that the first needle protection part 5.1 and the second needle protection part 5.2 are engaged or fitted to each other at the terminal DE side when the needle 31 is housed in the cover main body 2. As a result, the housed needle is prevented from being exposed outside the needle cover.

### BRIEF DESCRIPTION OF DRAWINGS

Fig. 1 is a perspective view of a needle cover of the present invention (a first embodiment of the present invention; the same applies to Figs. 2 to 5) viewed in a terminal DE direction and in a top U direction.
Fig. 2 is a perspective view of the needle cover of the present invention viewed in the terminal DE direction and in a bottom D direction.
Fig. 3 is a view of the needle cover of the present invention viewed in each of a first side S1 direction [see Fig. 3(A)], the bottom D direction [see Fig. 3(B)], the top U direction [see Fig. 3(C)], the terminal DE direction [see Fig. 3(E)], and a base PE direction [see Fig. 3(D)].
Fig. 4 is a view of the needle cover of the present invention viewed in each of the terminal DE direction and in the top U direction [see Fig. 4(A)] and the terminal DE direction and in the bottom D direction [see Figs. 4(B), 4(C)].
Fig. 5 is a view of the needle cover of the present invention viewed in each of the base PE direction and in the top U direction [see Fig. 5(A)] and the base PE direction and in the bottom D direction [see Fig. 5(B)].
Fig. 6 is a view of the needle cover of the present invention (a second embodiment of the present invention; the same applies to Figs. 7 to 8) viewed in each of the first side S1 direction [see Fig. 6(B)], the bottom D direction [see Fig. 6(A)], and the top U direction [see Fig. 6(C)].
Fig. 7 is a view of the needle cover of the present invention viewed in each of the terminal DE direction and in the top U direction [see Fig. 7(A)], the first side S1 direction and the top U direction [see Fig. 7(B)], the terminal DE direction and the bottom D direction [see Fig. 7(C)], and the base PE direction [see Fig. 7(D)].
Fig. 8 is a view of the needle cover of the present invention viewed in each of the base PE direction and in the bottom D direction [see Fig. 8 (A)], the base PE direction and in the top U direction [see Fig. 8(B)], the terminal DE direction and in the first side S1 direction [see Fig. 8(C)], the terminal DE direction [see Fig. 8(D)], and the base PE direction and in the top U direction [see Fig. 8(E)].
Fig. 9 is a view of the needle cover of the present invention (a third embodiment of the present invention; the same applies to Figs. 10 to 11) viewed in each of the first side S1 direction [see Fig. 9(B)], the bottom D direction [see Fig. 9(A)], and the top U direction [see Fig. 9(C)].
Fig. 10 is a view of the needle cover of the present invention viewed in each of the first side S1 direction and in the base PE direction [see Fig. 10(A)], the terminal DE direction and in the top U direction [see Fig. 10(B)], the terminal DE direction and in the bottom D direction [see Fig. 10(C)], and the base PE direction [see Fig. 10(D)].
Fig. 11 is a view of the needle cover of the present invention viewed in each of the first side S1 direction and in the bottom D direction [see Fig. 11 (A)], the base PE direction and in the U direction [see Fig. 11(B)], the first side S1 direction [see Fig. 11(C)], and the terminal DE direction [see Fig. 11(D)].
Fig. 12 is a schematic view of the needle cover of the present invention (the first embodiment; the same applies to Figs. 13 to 15) in a first state (a state before shielding a needle) viewed in the top U direction.
Fig. 13 is a schematic view of the needle cover of the present invention in a second state (a state after shielding the needle) viewed in the top U direction.
Fig. 14 is a schematic view of the needle cover of the present invention in a state of shifting from the first state to the second state (a shifting state) viewed in the first side S1 direction.
Fig. 15 is a schematic view of the needle cover of the present invention in the second state (the state after shielding the needle) viewed in the first side S1 direction.
Fig. 16 is a schematic view of the needle cover of the present invention (the third embodiment; the same applies to Fig. 17) in the state of shifting from the first state to the second state (the shifting or intermediate state) viewed in the first side S1 direction.
Fig. 17 is a schematic view of the needle cover of the present invention in the second state (the state after shielding the needle) viewed in the S1 direction.
Fig. 18 is a partially enlarged view of another example of Fig. 6 viewed in each of the first side S1 direction [see Fig. 18(B)] and the top U direction [see Fig. 18(A)].
Fig. 19 is a partially enlarged view of the first state of the third embodiment (Figs. 9 to 11) in the terminal DE direction and in the first side S1 direction.
Fig. 20 is a cross-sectional perspective view taken along A-A' of Fig. 19.
Fig. 21 is a cross-sectional view taken along A-A' of Fig. 19.
Fig. 22 is an enlarged view of the second state of the third embodiment (Figs. 9 to 11) in the terminal DE direction and in the first side S1 direction.
Fig. 23 is a cross-sectional perspective view taken along A-A' of Fig. 22.
Fig. 24 is a cross-sectional view taken along A-A' of Fig. 22.

### MODES FOR CARRYING OUT THE INVENTION

To facilitate understanding of a needle cover (1, 1', 1") of the present invention, the following definitions are given by using reference numerals used in claims, detailed description of the invention, etc. of the present invention.

The reference numerals used in claims and detailed description of the needle cover of the present invention are used for facilitating the understanding of the needle cover (1, 1', 1") of the present invention and the reference numerals themselves are not intended to specify or limit the scope of the invention.

The needle cover (1, 1', 1") of the present invention has a longitudinal L direction (or longitudinal direction L) and a side S direction (meaning a "short" direction relative to "longitudinal") approximately perpendicularly crossing the longitudinal L direction.

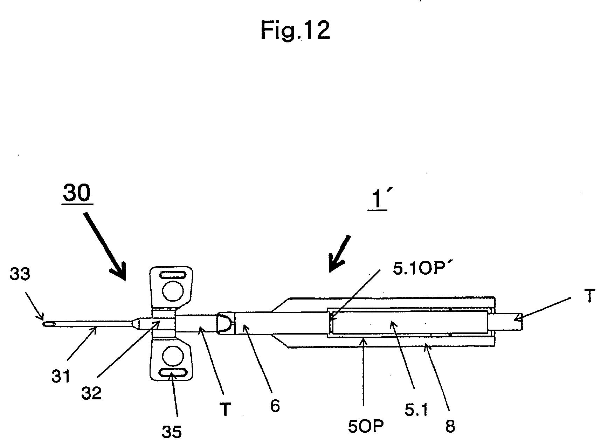

In the present invention, a "base PE (side or direction) " indicates one of the longitudinal L directions and means an end or terminal portion on a tube T side connected to a needle hub 32 as shown in Figs. 12, 13, 14, and 16.

A "terminal DE (side or direction)" indicates one of the longitudinal L directions and means an end or terminal portion on the side to which a needle 31 is attached as shown in Figs. 12, 13, 14, and 16.

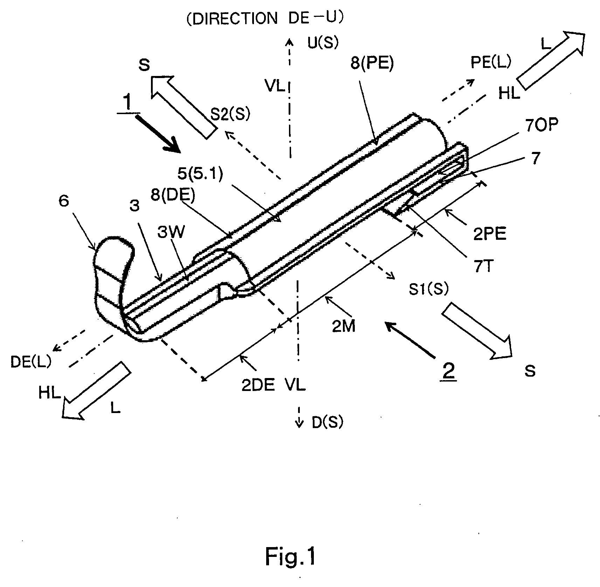

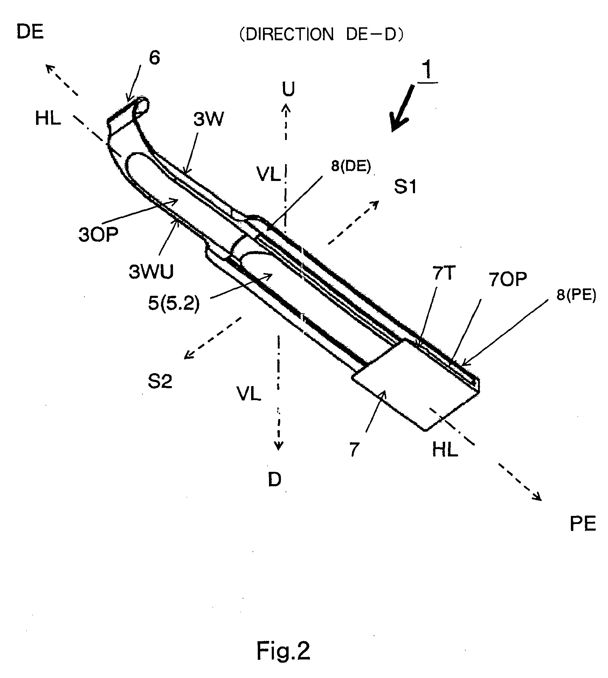

A "horizontal axis HL (direction)" means, for example, a direction extending in the longitudinal L direction of the needle cover 1 (also, 1', 1") as shown in Figs. 1 and 2 (see dashed lines in Figs. 1 and 2).

A "vertical axis VL (direction)" means, for example, a direction extending up and down in an up-down direction approximately vertically crossing the longitudinal L direction of the horizontal axis HL as shown in Figs. 1 and 2 (see dashed lines in Figs. 1 and 2).

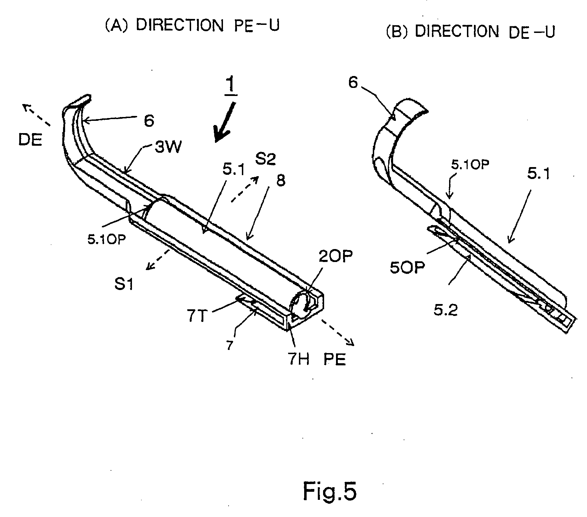

A "first side S1 (side or direction) " indicates one of the side S directions and means, for example, the direction of the right end of the needle cover 1 (also, 1', 1") viewed in the terminal DE direction as shown in Figs. 1 and 2 (see dashed arrows in Figs. 1 and 2). The "first side S1 (side or direction)" also means, for example, the direction of the left end of the needle cover 1 (also, 1', 1") viewed in the base PE direction as shown in Fig. 5 (see dashed arrows in Fig. 5).

A "second side S2 (side or direction)" indicates one of the side S directions and means, for example, the direction of the left end of the needle cover 1 (also, 1', 1") viewed from the terminal DE direction as shown in Figs. 1 and 2 (see dashed arrows in Figs. 1 and 2). The "second side S2 (side or direction)" also means, for example, the direction of the right end of the needle cover 1 (also, 1', 1") viewed from the base PE direction as shown in Fig. 5 (see dashed arrows in Fig. 5).

A "top U (side or direction)" indicates one of the side S directions and means, for example, the direction extending upward and approximately vertically crossing the longitudinal L direction of the horizontal axis HL as shown in Figs. 1 and 2 (see dashed lines in Figs. 1 and 2).

A "bottom D (side or direction) " indicates one of the side S directions and means, for example, the direction extending downward and approximately vertically crossing the longitudinal L direction of the horizontal axis HL as shown in Figs. 1 and 2 (see dashed lines in Figs. 1 and 2).

In the description of the present invention,
a "first state" is a state in which the needle cover 1 (also, 1', 1") is located in the base PE direction without shielding (protecting) the needle 31 as shown in Fig. 12, i.e., a state in which the needle is exposed before being shielded (protected) .

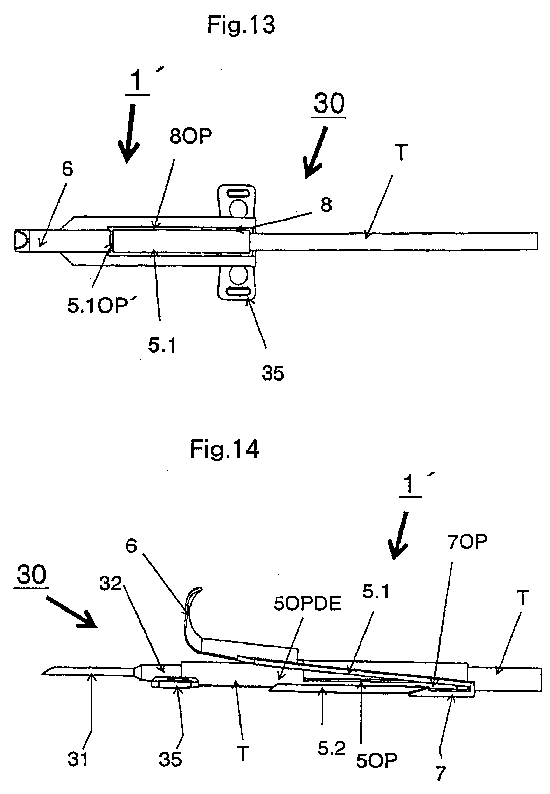

A "second state" is a state in which the needle cover 1 (also, 1', 1") is located in the terminal DE direction to shield (protect) the needle 31 as shown in Fig. 13, i.e., a state after the needle is shielded (protected).

A "shifting or transition state" means a state during a shift or transition from the "first state" to the "second state. " A "shift or transition start state" means an initial state of starting a shift or transition from the "first state" to the "second state."

A "shift or transition end state" means a state immediately before the termination of the shift or transition from the "first state" to the "second state."

### [Needle Cover (1, 1', 1")]

A first embodiment, a second embodiment, and a third embodiment will now be described as exemplary embodiments of the present invention although it is not intended to limit the scope of the present invention to these embodiments.

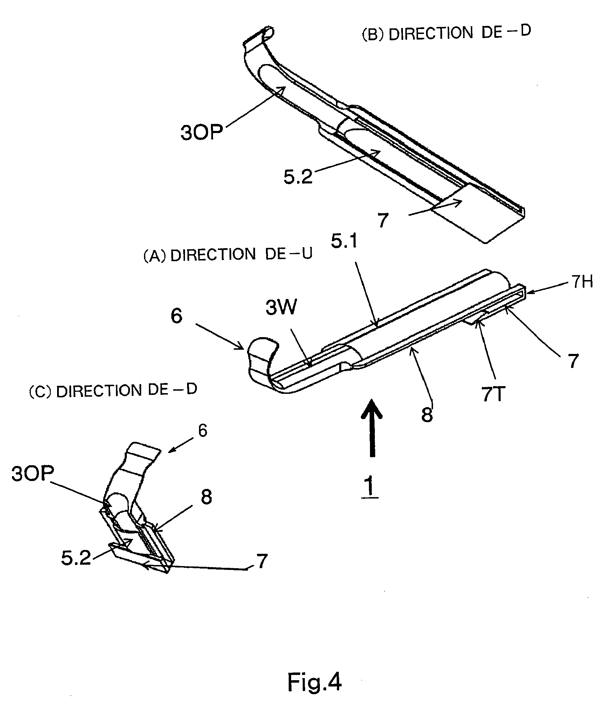

The needle cover 1 of the first embodiment of the present invention is shown in Figs. 1 to 5.

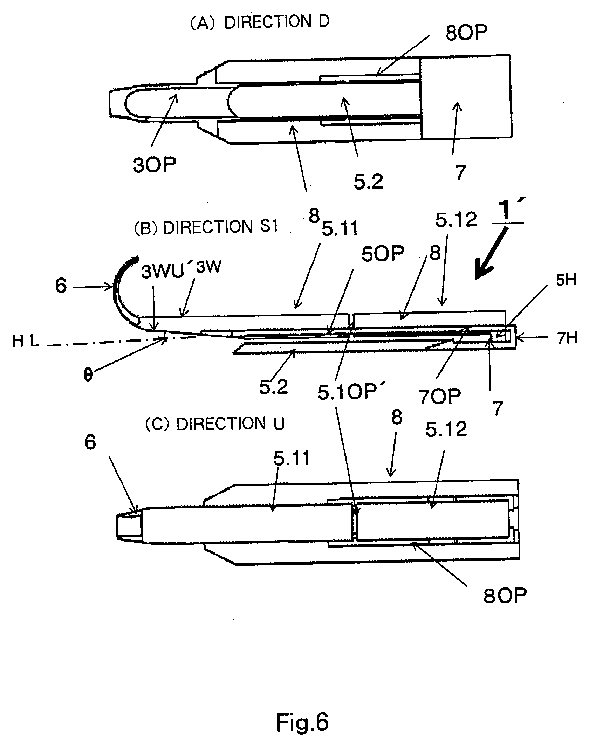

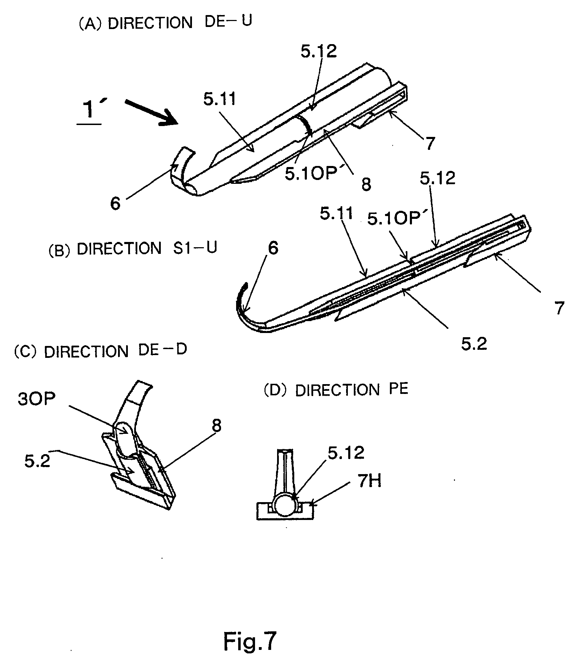

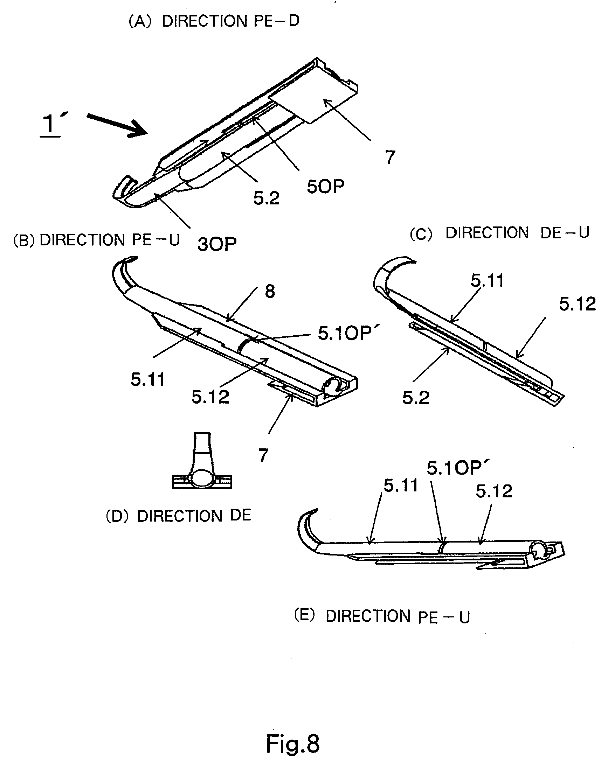

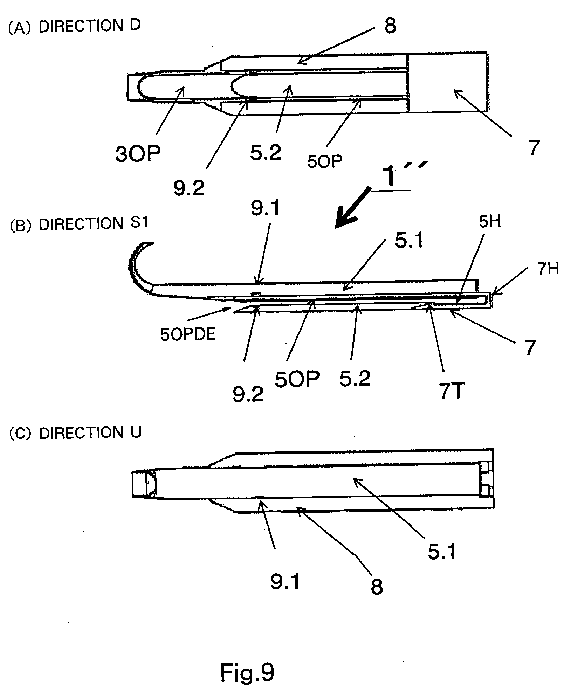

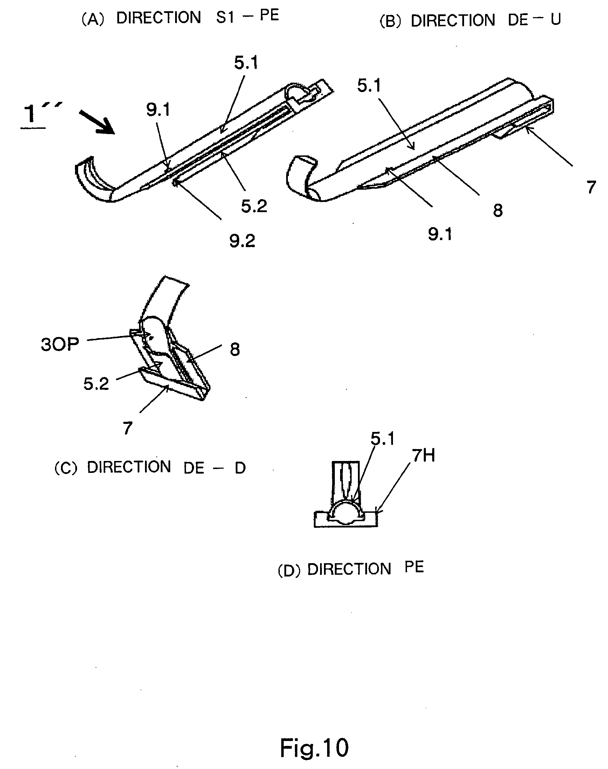

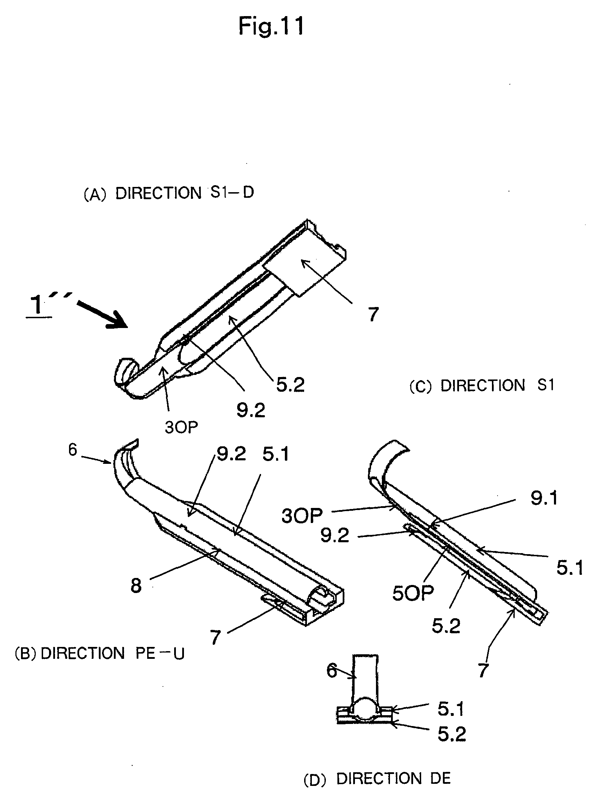

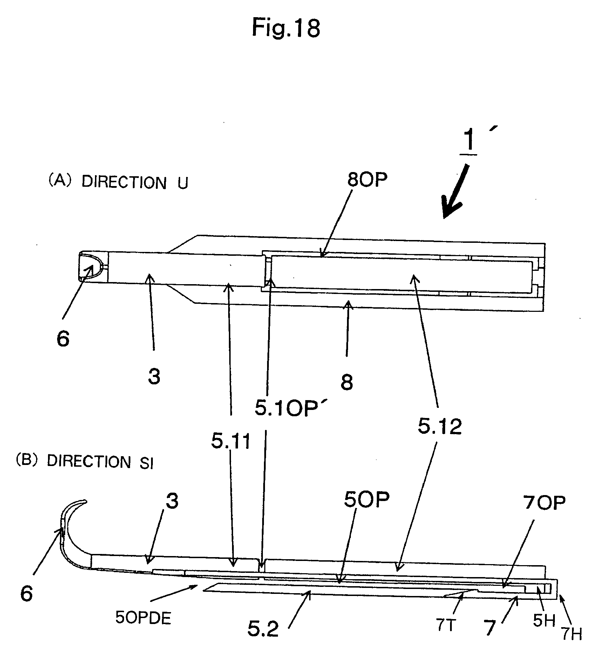

The needle cover 1' of the second embodiment is shown in Figs. 6 to 8 and Figs. 12 to 15. The needle cover 1" of the third embodiment is shown in Figs. 9 to 11 and Figs. 16 to 17. Fig. 18 is a partially enlarged view of Fig. 6 showing another example of the needle cover 1' of the second embodiment.

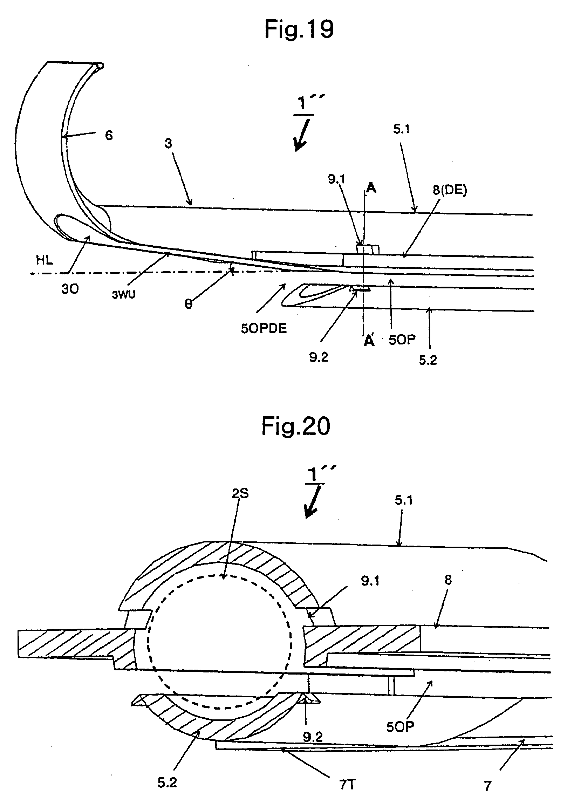

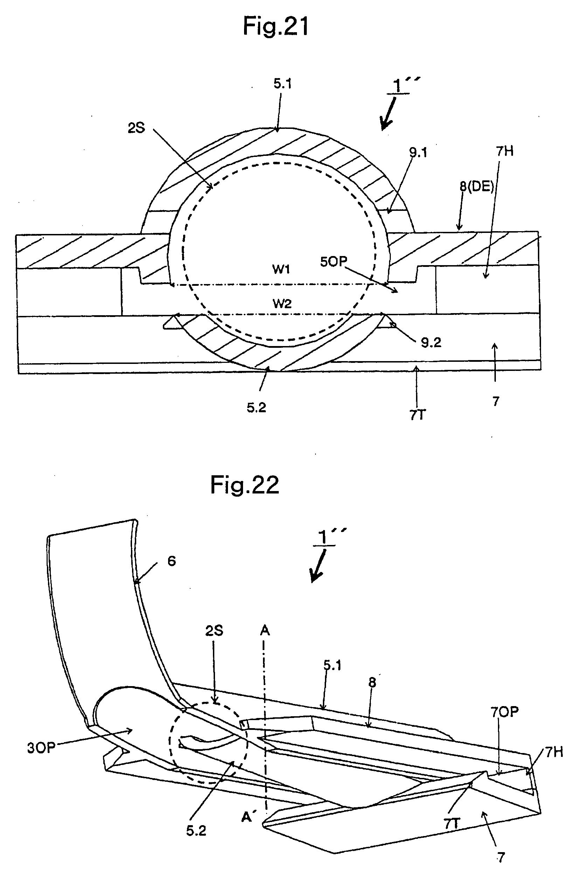

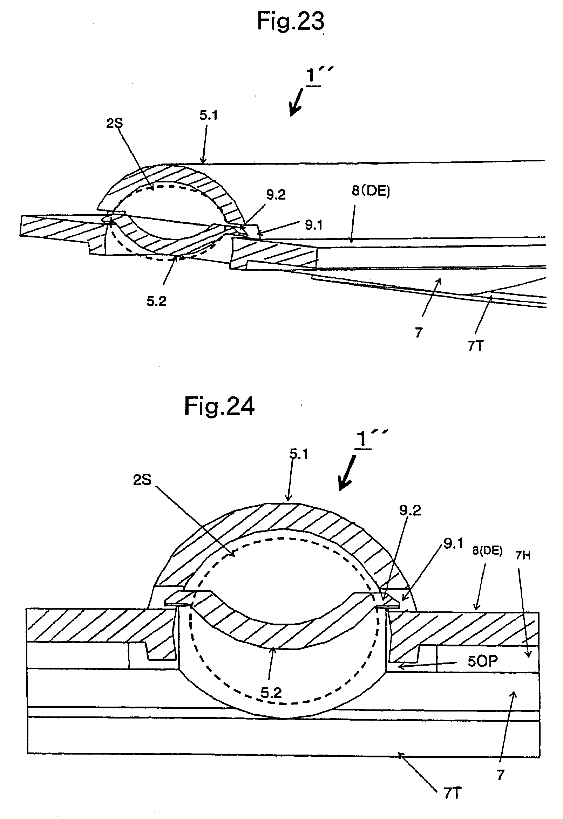

Figs. 19 to 21 are partially enlarged views of the first state of the needle cover 1" of the third embodiment, and Figs. 22 to 24 are enlarged views of its second state.

Description will hereinafter be given with reference to Figs. 1 to 24.

In the description of the needle cover 1' of the second embodiment and the needle cover 1" of the third embodiment, the same reference numerals are used for the members in common with, or substantially the same as, those of the needle cover 1 of the first embodiment in order to avoid the complication or redundancy of using excessively numerous reference numerals.

In the same manner, in the needle cover 1' of the second embodiment and the needle cover 1" of the third embodiment, the same reference numerals are also used for those components (members) which correspond to those of the needle cover 1 of the first embodiment when those components (members) are structurally different but have substantially the same or corresponding functions, to avoid the complexity of reference numerals.

### [Needle Cover 1 of First Embodiment]

As shown in Fig. 1, the needle cover 1 has a long-length cover main body 2 which has (or composed of) three parts, i.e., a terminal part (tip part) 2DE, an approximately middle part 2M, and a base part 2PE arranged and sectionalized in this order in the longitudinal L direction of the cover main body 2 (hereinafter also simply referred to as the "main body 2").

[(Note) In the description of the present invention, "having" (or forming) an "arbitrary member (or component)" as described above has substantially the same meaning as at least "having", "including", or "comprising" etc. the "arbitrary member (component)."]

A configuration of each of the sectionalized parts will hereinafter be described in more detail.

The needle cover 1 has a needle introduction part 3 on the terminal part 2DE side of the main body 2 as shown in Fig. 1.

The needle cover 1 has a needle protection part 5 over the approximately middle part 2M and the base part 2PE of the main body 2.

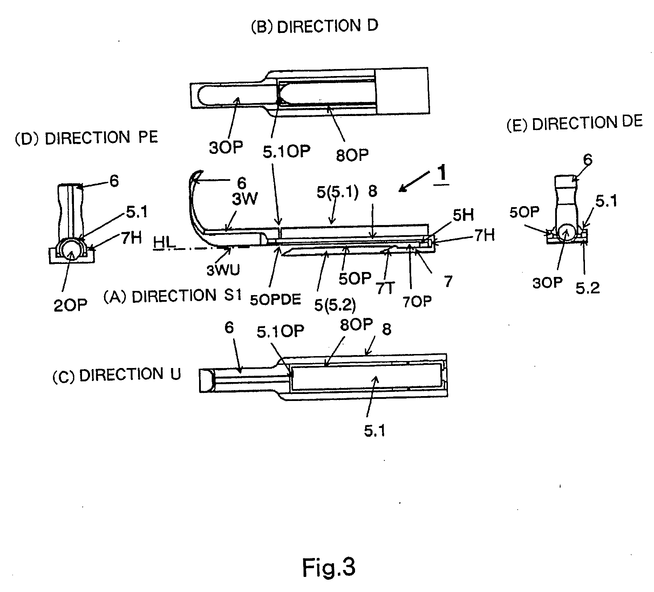

The needle cover 1 has (or forms) a base-side opening 20P on the base PE side of the base part 2PE of the main body 2 (see Fig. 3(D), Fig. 5(A), etc.). The base-side opening 20P is an opening from which a tube T is mainly pulled out. In particular, in the needle cover 1, the tube T is connected to the needle hub 32 of a winged needle 30 on the base PE side and is led outside through the base-side opening 20P from the needle cover 1.

[(Note) In the description of the present invention, "having" (or forming) an "opening" in an "arbitrary member (or component)" as described above means that at least one opening in an arbitrary shape may be formed at an arbitrary position in the "arbitrary member (or component)."]

### [Needle Introduction part 3]

For example, as shown in Figs. 1 to 2, the needle introduction part 3 is formed genarally in the terminal part 2DE of the main body 2 and has a wall 3W on the top U side and an opening 30P on the bottom D side.

The wall 3W and the opening 30P are each formed along the longitudinal L direction.

As shown in Figs. 2 and 3(A), a bottom wall 3WU of the wall 3W is formed approximately parallel to the horizontal axis HL. In particular, the bottom wall 3WU is formed in straight shape (also referred to as approximately in linear shape) in the longitudinal L direction without an inclination angle.

### [Needle Protection Part 5 (5.1, 5.2)]

The needle protection part 5 is a part for housing or accommodating the introduced needle to keep and prevent it from exposing, which has a first needle protection part (also referred to as a "top protection part" or "top first needle protection part") 5.1 on the top U side and a second needle protection part (also referred to as a "bottom protection part" or "bottom second needle protection part") 5.2 on the bottom D side, as shown in Fig. 3(A), for example.

The needle protection part 5 has a groove-shaped or slot-shaped opening 50P in the longitudinal L direction as shown in Figs. 3(A), 5(B), 6(B), 8(A), etc., between the bottom D side of the first needle protection part (top protection part) 5.1 and the top U side of the second needle protection part (bottom protection part) 5.2 (in this regard, the opening 5OP may be referred to as a groove or a slot).

As shown in Figs. 3(A) and 6(B), the first needle protection part (top protection part) 5.1 and the second needle protection part (bottom protection part) 5.2 are connected at a base PE side end by a needle protection part connection unit 5H.

As shown in Figs. 3(A), 14, etc., the first needle protection part 5.1 and the second needle protection part 5.2 have or form a terminal opening 50PDE at a terminal DE side end.

In the illustration of Figs. 1 to 5, the first needle protection part (top protection part) 5.1 is formed in a so-called "approximately semi-cylindrical shape". The second needle protection part (bottom protection part) 5.2 is formed in a so-called "approximately plate shape."

The first needle protection part 5.1 is formed from the terminal opening 50PDE of the needle protection part 5 (Figs. 3(A) and 14) in the longitudinal L direction to the base part 2PE side end of the main body 2.

The second needle protection part 5.2 is formed from the terminal opening 50PDE of the needle protection part 5 in the longitudinal L direction to the terminal DE side of a wing-fixing unit 7 (described below).

The second needle protection part 5.2 and the wing-fixing unit 7 is connected by a wing-fixing unit connection unit 7H on the base PE side (Figs. 3(A), 6(B), and 7(D)).

The shapes or structures of the first needle protection part (or top protection part) 5.1 and the second needle protection part (or bottom protection part) 5.2 are not limited to the "approximately semi-cylindrical shape" and the "approximately plate shape" as shown in Figs. 1 to 5 and may assume any shapes as long as the objects of the present invention can be achieved.

### [Opening 50P]

The opening 50P is a place (or gap) allowing passage of wings 35 of the winged needle 30 (see Figs. 3(A), 5(B), 6(B), 8(A), 14, etc.).

The width of the opening 50P (i.e., the size of the gap) is made substantially the same, from the terminal opening 50PDE of the needle protection part 5 in the longitudinal L direction to the terminal DE side of the wing-fixing unit 7.

This enables the wings 35 of the winged needle 30 to move smoothly through the opening 50P (or gap) without rattling when the wings 35 make a move from the terminal opening 50PDE to the wing-fixing unit 7.

### [Gap 5.10P]

As shown in Fig. 3, a slight or small gap 5.10P (also referred to as a slit or an opening) is formed between the base PE side of the needle introduction part 3 and the terminal DE side of the first needle protection part (top protection part) 5.1 of the needle protection part 5.

Thus is the gap 5.10P formed in this way, the base PE side of the needle introduction part 3 can be moved separated from the terminal DE side of the first needle protection part 5.1 at the step of the "shift (or transition) start state" (meaning the initial state at which starting of a shift from the "first state" to the "second state" begins.).

Because of the presence of the gap 5.1OP, the terminal opening 50PDE (Fig. 3(A)) of the needle protection part 5 can be expanded widely, which facilitates the winged needle 30 to be more easily housed or accommodated in the needle cover 1.

### [Needle Protection Part Connection Unit 5H] (Figs. 3 (A) and 6(B))

The needle protection part connection unit 5H is a part acting as a so-called "hinge" when the (needle protection part) opening 50P and a (wing-fixing unit) opening 70P are expanded open toward the top U side and the bottom D side at the "shift start state" (the initial or starting state of a shift from the "first state" to the "second state") or the "transition state" (the intermediate state from the "first state" to the "second state").

### [Leg 8]

The first needle protection part (top protection part) 5.1 has a pair of legs 8 separated by a gap 80P (also referred to as a slit or an opening) and formed on the bottom D side in the longitudinal L direction as well as the first side S1 and second side S2 directions of the needle cover 1 from the terminal DE side to the base part 2PE (see, e.g., Figs. 3A, 5(A), and 6(B)).

[(Note) In the description of the present invention, "forming" "another member (or component)" at one end of an "arbitrary member (or component)" as described above, has substantially the same meaning as "connecting", "coupling", "extending", "projecting", or "disposing", etc.]

As shown in the figures described above (Figs. 3A, 5(A), and 6(B)), on the base PE side, the legs 8 are connected to the wing-fixing unit 7 via the wing-fixing unit connection unit 7H on the bottom D side of the base part 2PE.

In the thus described manner, the legs 8 are, on the terminal DE side, connected to the base PE side of the needle introduction part 3 and on the base PE side, connected to the base PE side of the wing-fixing unit 7.

The legs 8 allow the needle introduction part 3 (the base PE side of it) to be moved separated from (the terminal DE side of) the first needle protection part 5.1 (or top protection part) and to be pulled up in the top U direction, together with a handle 6 and the gap 5.1OP, when the step of "shift start state" (the initial state of starting a shift from the "first state" to the "second state")is made (i.e., thus the legs 8 provide support when the needle introduction part 3 is pulled up in the top U direction).

As a result, the terminal opening 50PDE of the needle protection part 5 is expanded widely, facilitating the winged needle 30 to be easily housed or accommodated in the needle cover 1 (see Figs. 3(A), 14, etc.).

### [Wing-Fixing Unit Connection unit 7H]

As shown in Fig. 3 (A) etc., the wing-fixing unit connection unit 7H acts as a so-called "hinge" composed of the second needle protection part (bottom protection part) 5.2 and the wing-fixing unit 7 when the (needle protection part) opening 5OP and the (wing-fixing unit) opening 70P are made open toward the top U side and the bottom D side at the "shifting state" (or the intermediate state from the "first state" to the "second state") or at the "shift or transition end state" (the state immediately before the end of the shift from the "first state" to the "second state").

### [Handle 6]

As shown in Fig. 1 etc. , the needle cover 1 has the handle 6 positioned on the most distal end of the terminal DE side relative to the terminal part 2DE of the main body 2.

The handle 6 is formed in an approximately plate shape (or also referred to as an approximately curved plate shape or an approximately belt shape).

The handle 6 has a bottom D side end connected to the terminal DE side end of the wall 3W of the needle introduction part 3 and is formed in a shape erecting upwardly toward the top U side from the horizontal axis HL (an approximately horizontal position) shown in Fig. 1.

When in use, the handle 6 is held on the top U side and pulled upwardly toward the top U side to pull up the needle introduction part 3 in the top U direction, and thereby the terminal opening 50PDE is expanded and wide-open, thus allowing the winged needle 30 to be easily housed or accommodated in the needle cover 1. The shape or structure of the handle 6 is not limited to the shape shown in the figures given here and may have any shapes as long as the objects of the present invention can be achieved.

### [Wing-Fixing Unit 7]

The needle cover 1 also has the wing-fixing unit 7 in the base part 2PE.

The wing-fixing unit 7 is connected at the base PE side end to the bottom D side ends of the legs 8PE.

The wing-fixing unit 7 is connected at the base PE side to the wing-fixing unit connection unit 7H, and also connected to the base PE side end of the second needle protection part (bottom protection part) 5.2.

The wing-fixing unit 7 also has a locking part 7T formed at the terminal DE side end on the both sides (or the first side S1 and the second side S2) of the second needle protection part (or bottom protection part) 5.2. The locking part 7T is formed in a so-called "wedge shape" (see Figs. 1 to 5).

### [Opening 70P]

The opening 70P is a space formed between the top U side of the wing-fixing unit 7 and the bottom D side of the first needle protection part (top protection part) 5.1. The opening 70P is formed continuously to the base PE side of the opening 50P (see Figs. 1 to 3). The opening 70P is provided for the purpose of housing or accommodating the wings 35 passing through the opening 50P to be fixed there, for wing-fixing, with the wings 35 being locked to the locking part 7T.

The opening 70P may be referred to as a space, a groove, a slot, etc., because of, or according to the shape thereof.

In Fig. 3, the width (size) of the opening 70P is made substantially the same as the width (size) of the opening 50P.

Thus forming the opening 70P in this way, the wings 35 are facilitated to be easily fixed to the wing-fixing unit 7 after the wings 35 pass through the first needle protection part (or top protection part) 5.1.

Moreover, since the width or gap of the opening 50P is made substantially the same over the length from the terminal DE side to the base PE side, the wings 35 can be guided through this opening stably (or without causing rattling) to the wing-fixing unit 7 and is fixed there.

The width or gap of the opening 50P may be made substantially the same size at least over the length from the terminal DE side to the terminal DE of the opening 70P of the wing-fixing unit 7. The opening 70P of the wing-fixing unit 7 may be formed wider (larger) than the width (size) of the opening 5OP.

Even after the wings 35 are once fixed, it might occur that the thus fixed wings 35 come off when the first needle protection part (or top protection part) 5.1 on the base side is allowed to open, therefore, the first protection part 5.1 on the base PE side is so configured as to retain the same shape with the constant-sized gap 50p.

### [Needle Cover 1' of Second Embodiment]

The needle cover 1' of the second embodiment is as exemplarily illustrated in Figs. 6 to 8, 12, and 17 to 18.

The needle cover 1' has the following differences from the needle cover 1 of the first embodiment:
First, as shown in Fig. 6, the needle cover 1' has a gap 5.1OP' formed at a position of the approximately middle part of the first needle protection part (or top protection part) 5.1 and is divided into a terminal side first needle protection part 5.11 (top U and terminal DE side) and a base side first needle protection part 5.12 (top U and base PE side) in the side (or first side S1, second side S2) direction.

As shown in Fig. 18, the gap 5.10P' may be formed close to (or in the vicinity of) the needle introduction part 3 of the first needle protection part 5.1.

Instead, in this case, the base PE side end of the needle introduction part 3 is connected to the terminal side first needle protection part 5.11 (on the top U and terminal DE side).

As described above, at the "shift start state" step (or the initial state of starting a shift from the "first state" to the "second state"), the base PE side of the terminal DE side first needle protection part (or top protection part) 5.11 (on the top U and terminal DE side) (connected to the base PE side end terminal side of the needle introduction part 3) can be moved separated from the terminal DE side of the base side first needle protection part 5.12 (on the top U and base PE side).

As a result, a first feature is that since the terminal opening 50PDE of the needle protection part 5 and the opening 50 (of the needle protection part 5) can be expanded further wider (as compared to the case of the needle cover 1 of the first embodiment) at the "shift start state" or the "shifting or transition state" step, thus, an advantageous effect of further facilitating the housing or accommodating of the winged needle 30 in the needle cover 1 is produced.

A second feature is that a bottom wall 3WU' of the wall 3W is inclined upward in the terminal DE direction relative to the horizontal axis HL as shown in Fig. 6 (also in Fig. 18). In other words, the bottom wall 3WU' is formed to have an inclination angle θ.

The bottom wall 3WU' being thus composed of the upwardly-inclined constitution, in the "shift start state" step and the "shift transition state" step, the winged needle 30 can more easily be housed or accommodated in the needle cover 1 from the opening 30 of the needle introduction part 3 (as compared to the case of the needle cover 1 of the first embodiment).

### [Needle Cover 1" of Third Embodiment]

The needle cover 1" of the third embodiment is as exemplarily illustrated in Figs. 9 to 11, 16-17, 19-21, and 22 to 24.

The needle cover 1" has the following differences from the needle cover 1 of the first embodiment and the needle cover 1' of the second embodiment:
The first needle protection part (or top protection part) 5.1 and the second needle protection part (or bottom protection part) 5.2 have widths made different from each other in the side (first side S1, second side S2) direction.

In particular, as shown in Figs. 19 to 21, a diameter (maximum width in the side S direction) W2 of the second needle protection part 5.2 is made smaller than a diameter (minimum width in the side S direction) W1 of the first needle protection part 5.1 (see Fig. 21). In other words, the diameter (minimum width in the side S direction) W1 of the first needle protection part (or top protection part) 5.1 is made larger than the diameter (maximum width in the side S direction) W2 of the second needle protection part (or bottom protection part) 5.2.

Thus constituted, after the needle 31 is housed in the cover main body 2, as shown in Figs. 22 to 24, the terminal DE side of the second needle protection part (or bottom protection part) 5.2 can be further moved upward till it is to be housed and forced into a space part 2S on the terminal DE side of the first needle protection part (or top protection part) 5.1, and thus, the needle 31 of the winged needle 30 can be clamped and held more firmly between the first needle protection part 5.1 and the second needle protection part 5.2, and also between the top U side and the bottom D side and between the first side S1 and the second side S2.

Moreover, to maintain the state of holding by "clamping, " as shown in Figs. 9 and 19 to 20, the needle cover 1" forms an engaging part 9 composed of the first needle protection part (or top protection part) 5.1 and the second needle protection part (or bottom protection part) 5.2.

More specifically, the engaging part 9 is composed of a first engaging part (or top U side) 9.1 formed on the first needle protection part 5.1 and a second engaging part (or bottom D side) 9.2 formed on the second needle protection part 5.2.

In the illustration of Figs. 19 and 20 (partially enlarged views), the engaging part 9 has a so-called "hole (through-hole) " formed in the first needle protection part (top protection part) (top U side) 5.1 as the first engaging part (top U side) 9.1 and has a so-called "projection" (or "protrusion") on the second needle protection part (or bottom protection part) (bottom D side) 5.2 as the second engaging part (bottom D side) 9.2.

In particular, the "projection" acting as the second engaging part (the bottom D side) 9.2 is formed on the terminal DE side of the second needle protection part 5.2 and is formed (projected) on the second needle protection part 5.2 top U side as well as first side S1 and second side S2.

The "hole(through-hole)" acting as the "first engaging part (U side) 9.1" is formed in the vicinity of the legs 8 on the bottom D side of the first needle protection part (U side) 5.1.

The needle cover 1" is so configured as described above that it is therefore characterized in that formation of the gap 80P (also referred to as a slit or an opening) may not be required, which is, as in the covers (1, 1') of the first embodiment and second embodiment, formed between the legs 8 and the main body 2 from the terminal DE side of the approximately middle part 2M to the base side of the base part 2PE. The needle cover 1" of the third embodiment is also characterized in that the gap 5.1OP (Fig. 3) and 5.1OP' (Fig. 6) may not be requested to form, as were necessary in the covers (1, 1') of the first and second embodiments.

### [Winged Needle 30 and Handling Thereof]

The winged needle 30 used in the present invention has the needle 31 and the approximately tubular needle hub 32 (see Fig.12).

As exemplarily illustrated in Fig. 12, the needle 31 has a narrow tubular shape and is formed approximately linearly.

The needle 31 has a sharp terminal end 33 and the base PE side of which is fixed (attached) to the terminal DE side of the needle hub 32.

The terminal end 33 of the needle 31 punctures a patient and a drug solution etc. is injected into the patient via the terminal end 33 through the tube T connected to the base PE side of the needle hub 32.

After the injection of drug solution into the patient is finished, the needle 31 is pulled out from the patient.

### [First State]

### (Assembling of Needle Cover 1 and Winged Needle 30; Preparatory Phase)

The most typical needle cover 1' of the second embodiment will hereinafter be described mainly with reference to the drawings thereof (the needle cover 1 of the first embodiment and the needle cover 1" of the third embodiment will be described, touching only on the different portions, along the way or at the end of the description; substantially, the same portions will not be described).

First, the tube T connected to the base PE side of the needle hub 32 is inserted into the main body 2 of the needle cover 1' from its terminal DE side to the base PE side. In usual practice, a cap (not shown) is attached to the needle 31 before use or during storage for the purpose of prevention of erroneous puncture. (Although, the term "cap" may occasionally be referred to as a "cover", in this description, the term "cap" (instead of "cover") is used deliberately to distinguish it from the specific "needle cover" of the present invention for preventing the erroneous puncture of the needle after use.)

As a result, the "first state" is achieved as shown in Fig. 12. In particular, the winged needle 30 is on the terminal DE side and the needle cover 1' is on the base PE side. The needle 31 is not shielded (protected) by the needle cover 1'. This state is a "preparatory phase".

### [Protection (Shielding) of Terminal End 33 of Needle 31]

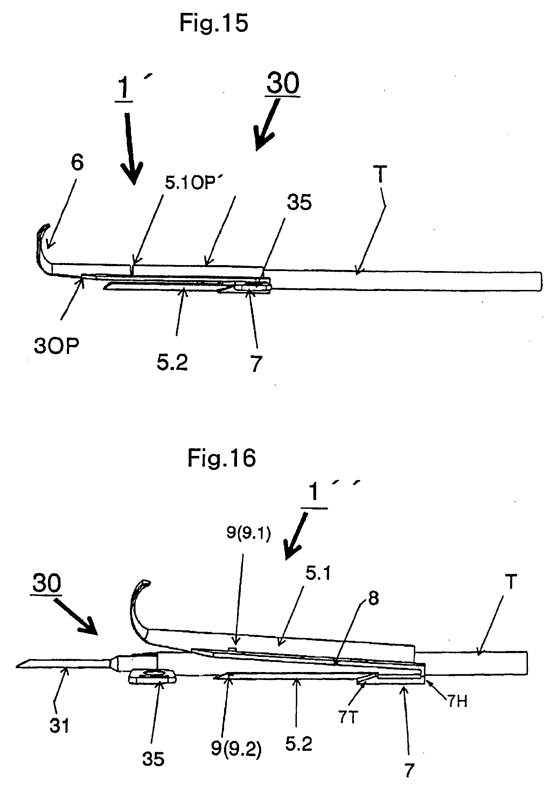

<1> [Shift Start State]
   While the needle cover 1' is held by one hand, the tube T is pulled by the other hand toward the base PE side.
<2> The base PE side of the tube T and the needle hub 32 are drawn from the opening 30P of the needle introduction part 3 into the needle cover 1' (or the main body 2).
<3> Immediately before the wings 35 are subsequently moved to the vicinity of the terminal opening 50PDE, the handle 6 is pulled up toward the top U side, and the base PE side of the terminal side first needle protection part (or top protection part) 5.11 is moved and separated (together with the needle insertion part 3) from the terminal DE side of the base side first needle protection part (or top protection part) 5.12 and is moved toward the top U side together with the terminal DE side of the legs 8.
   [(Note) In the case of the needle cover 1 of the first embodiment, the base PE side of the needle introduction part 3 is moved separated from the terminal DE side of the first needle protection part (top protection part) 5.1 and is moved toward the top U side together with the terminal DE side of the legs 8.)]
   The terminal opening 50PDE is opened wider as shown in Fig. 14 [as compared to Fig. 3(A)].
<4> As the tube T is further pulled toward the base PE side, the opening 50P (of the needle protection part 5) are, and the opening 70P (of the wing-fixing unit 7) are, respectively opened by using the needle protection part connection unit 5H (Fig. 6(B)), and the wing-fixing unit connection unit 7H, as a starting points of a hinge toward the top U side and the bottom D side in conjunction with each other. This leads to a state in which the winged needle 30 is more easily housed or accommodated in the needle cover 1'. The wings 35 move through the opening 50P toward the base PE side.
<5> [Shift End State]
   When the tube T is further pulled toward the base PE side, the wings 35 enter the opening 70P. The needle 31 is housed in the needle protection part 5 [comprising terminal side first needle protection part (or top protection part) 5.11, the base side first needle protection part (or top protection part) 5.12, and the second needle protection part (or bottom protection part) 5.2]. [(Note) In the needle cover 1 of the first embodiment, needle protection part is made up of the first needle protection part (or top protection part) 5.1 and the second needle protection part (or bottom protection part) 5.2.] (More specifically, in this case, the needle 31 is housed in a space formed by the first needle protection part 5.1 and the second needle protection part 5.2)
<6> [Second State]
   The handle 6 is put down toward the bottom D side and returned to the original position. The needle 31 is housed in the needle protection part 5 [comprising the terminal side first needle protection part (or top protection part) 5.11, the base side first needle protection part (or top protection part) 5.12, and the second needle protection part (or bottom protection part) 5.2]. [(Note) In the case of the needle cover 1 of the first embodiment, needle protection part 5 is made up of the first needle protection part (or top protection part) 5.1 and the second needle protection part (or bottom protection part) 5.2].
<7> The wings 35 are fixed by the locking part 7T as shown in Fig. 15 and the needle 31 is protected (or shielded) by the needle protection part 5 [comprising the terminal side first needle protection part (or top protection part) 5.11, the base side first needle protection part (or top protection part) 5.12, and the second needle protection part (or bottom protection part) 5.2]. [(Note) In the case of the needle cover 1 of the first embodiment, needle protection part is made up of the first needle protection part (or top protection part) 5.1 and the second needle protection part (or bottom protection part) 5.2.]

As shown in Figs. 13 and 15, the wings 35 are fixed to the wing-fixing unit 7 and the needle 31 is protected (or shielded) by the needle protection part 5 [comprising the terminal side first needle protection part 5.11, the base side first needle protection part 5.12, and the second needle protection part 5.2], thus is constituted the "second state". [(Note) In the case of the needle cover 1 of the first embodiment, needle protection part 5 is made up of the first needle protection part 5.1 and the second needle protection part 5.2.]

In the second state, the wings 35 are firmly fixed to the wing-fixing unit 7 and therefore prevented from moving in the terminal DE direction, thus never to be exposed outside the needle cover 1'.

### (Case of Third Embodiment)

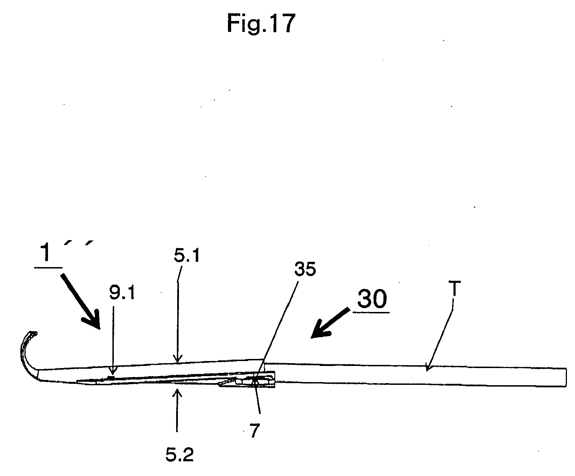

In the case of the needle cover 1" of the third embodiment, as shown in Figs. 16, 17, and 22 to 24 (the winged needle is not shown), the following steps of operations are performed, instead of the second embodiment, for [Shift Start State] step of [Protection (Shielding) of Terminal End 33 of Needle 31]:
<1> The base PE side of the tube T and the needle hub 32 are drawn from the opening 30P of the needle introduction part 3 into the needle cover 1" (the main body 2).
<2> Immediately before the wings 35 are subsequently moved to the vicinity of the terminal opening 50PDE, the handle 6 is pulled up toward the top U side, and the first needle protection part (top protection part) 5.1 is moved separated from the terminal DE side toward the top U side.
   The terminal opening 50PDE is wide opened as shown in Fig. 16.
<3> As the tube T is further pulled toward the base PE side, the opening 50P (of the needle protection part 5) and the opening 70P (of the wing-fixing unit 7) are, respectively opened, by using the needle protection part connection unit 5H, or by using the wing-fixing unit connection unit 7H, each as a starting point of a hinge toward the top U side and the bottom side in conjunction with each other. This leads to a state in which the winged needle 30 is more easily housed or accommodated in the needle cover 1". The wings 35 move through the opening 50P toward the base PE side.
<4> [Shift End State]
   When the tube T is further pulled toward the base PE side, the wings 35 enter the opening 70P. The needle 31 is housed in the needle protection part 5 (comprising the first needle protection part (top protection part) 5.1 and the second needle protection part (bottom protection part) 5.2).
<5> [Second State]
   The terminal DE side of the second needle protection part (bottom protection part) 5.2 is moved and housed into the space part 2S on the terminal DE side of the first needle protection part (top protection part) 5.1 to clamp the needle 31 of the winged needle 30 between the first needle protection part (or top protection part) 5.1 and the second needle protection part (or bottom protection part) 5.2, and thus, the needle 31 being locked between the top U side and the bottom D side and between the first side S1 and the second side S2.
   More specifically, the first engaging part on the first needle protection part (top protection part) 5.1 side is engaged with the second engaging part 9. 2 on the second needle protection part (bottom protection part) 5.2 side.
<6> The wing-fixing unit 7 is fixed by the locking part 7T and the needle 31 is protected (shielded) by the needle protection part 5 [comprising the first needle protection part (top protection part) 5.1 and the second needle protection part (bottom protection part) 5.2]. (More specifically, the needle 31 is housed and accommodated in a space formed by the first needle protection part 5.1 and the second needle protection part).
<7> As shown in Fig. 17, the wings 35 are fixed to the wing-fixing unit 7 and the needle 31 is protected (shielded) by the needle protection part 5 (comprising the first needle protection part 5.1 and the second needle protection part 5.2), thus constituting the "second state."

When the first engaging part 9.1 of the first needle protection part (top protection part) 5.1 is engaged with the second engaging part 9.2, the second needle protection part (bottom protection part) 5.2 is pushed into the space part 2S of the main body 2 and the needle 31 can be clamped and fixed between the first needle protection part 5.1 and the second needle protection part 5.2. The needle 31 can therefore be prevented more reliably from moving in the terminal DE direction and never be exposed outside the needle cover 1".

In the second state, thus, since the wings 35 are firmly fixed to the wing-fixing unit 7, the needle 31 is prevented from moving together with the wings 35 in the terminal DE direction and never be exposed outside the needle cover 1".

### (Materials to form Needle Cover)

The needle cover (1, 1', 1") of the present invention is preferably formed by integral molding from the same material.

For example, the needle cover is preferably made of an injection-moldable thermoplastic synthetic resin and examples thereof include, but not limited to, PE (polyethylene), PP (polypropylene), PB (polybutylene), PS (polystyrene), PVC (polyvinyl chloride), PMMA (polymethyl methacrylate), PMA (polymethyl acrylate), PC (polycarbonate), and PAm (polyamide), for example.

### INDUSTRIAL APPLICABILITY

As described above, in the needle cover of the winged needle of the present invention the needle protection part 5 (the first needle protection part 5.1 and the second needle protection part 5.2) and the wing-fixing unit 7 are separately formed and configured, which can achieve the stable fixation of the wings 35. And, since the needle cover of the present invention has the needle protection part 5 divided into two parts, i. e., the first needle protection part 5.1 and the second needle protection part 5.2, which allows, when the winged needle 30 is drawn into the cover main body 2, the wings 35 are easily inserted with significantly improved manipulability, thus the needle cover of the present invention can preferably be used in actual medical fields.

### EXPLANATIONS OF LETTERS OR NUMERALS

1, 1', 1" needle cover
2 cover main bode (main body)
2DE terminal part
2M approximately middle part
2PE base part
2S space part
20P base-side opening
3 needle introduction part
30P opening
3W wall
3WU bottom wall
5 needle protection part
5.1 first needle protection part (also referred to as "top protection part" or "top first needle protection part") (top U side)
5.11 terminal side first needle protection part (top U/terminal DE side)
5.10P gap
5.10P' gap
5.12 base side first needle protection part (top U and base PE side)
5.2 second needle protection part (also referred to as "bottom protection part" or "bottom second needle protection part") (bottom D side)
5OP opening
50PDE terminal side opening
5H needle protection part connection unit
6 handle
7 wing-fixing unit
70P opening
7T locking part
7H wing-fixing unit connection unit
8 leg
80P gap
8DE (terminal side) leg
8PE (base side) leg
9 engaging part
9.1 first engaging part (top U side)
9.2 second engaging part (bottom D side)
L longitudinal direction
HL horizontal axis
VL vertical axis
PE base
DE terminal
S side direction
S1 first side
S2 second side
U top
D bottom
30 winged needle
31 needle
32 needle hub
33 terminal end
35 wing
T tube

## Claims

1. A needle cover (1, 1', 1") housing and protecting a winged needle after its use, the needle cover (1, 1', 1") having: a main body (2) and having a terminal part (2DE), an approximately middle part (2M), and a base part (2PE) in a longitudinal (L) direction of the main body (2),
the main body (2) having a needle introduction part (3) on the terminal part (2DE) side, the needle introduction part (3) having an opening (30P) formed on a bottom (D) side, the main body (2) having a base-side opening (20P) on the base part (2PE) side,
the main body (2) having a needle protection part (5) over the approximately middle part (2M) and the base part (2PE), the needle protection part (5) having a first needle protection part (5.1) on a top (U) side and having a second needle protection part (5.2) on a bottom (D) side,
the needle cover (1, 1', 1") having an opening (5OP) formed between the bottom (D) side of the first needle protection part (5.1) and the top side of the second needle protection part (5.2),
the first needle protection part (5.1) and the second needle protection part (5.2) being connected on the base (PE) side by a needle protection part connection unit (5H) and having a terminal opening (50PDE) on the terminal (DE) side,
the second needle protection part (5.2) having a wing-fixing unit (7) formed on the base (PE) side and also as a first side (S1) and a second side (S2),
the wing-fixing unit (7) having a locking part (7T) formed on the terminal (DE) side.

2. The needle cover (1, 1', 1") according to claim 1, wherein
the needle cover (1, 1', 1") has an opening (70P) formed between the top (U) side of the wing-fixing unit (7) and the bottom (D) side of the first needle protection part (5.1), and wherein the opening (70P) is formed continuously to the base (PE) side of the opening 5OP.

3. The needle cover (1, 1', 1") according to claim 1 or 2, wherein
the opening (5OP) has the width or gap size made substantially the same from the terminal (DE) side to the base (PE) side.

4. The needle cover (1, 1') according to any one of claims 1 to 3, wherein
the first needle protection part (5.1) has a pair of legs (8) separated by a gap (80P) and formed on the bottom (D) side in the longitudinal (L) direction as well as the first side (S1) and second side (S2) directions of the needle cover (1) from the terminal (DE) side to the base part (2PE), wherein
the legs (8) are connected to the wing-fixing unit connection unit (7H) on the base (PE) side as well as the bottom (D) side of the base part (2PE) of the main body (2), and wherein
the legs (8) are connected to a base (PE) side end of the needle introduction part (3) at a terminal (DE) side end of the main body (2).

5. The needle cover (1) according to any one of claims 1 to 4, wherein
a gap (5.10P) is formed between the terminal (DE) side of the first needle protection part (5.1) and the base (PE) side of the needle introduction part (3).

6. The needle cover (1') according to any one of claims 1 to 4, wherein
a gap (5.1OP') is formed in the approximately middle part of the first needle protection part (5.1), and wherein
a terminal side first needle protection part (5.11) and a base side first needle protection part (5.12) are formed.

7. The needle cover (1") according to any one of claims 1 to 6, wherein
at least on the terminal (DE) side, the second needle protection part (5.2) has a width (W2) in the side (S) direction made smaller than a width (W1) in the side (S) direction of the first needle protection part (5.1), wherein
a pair of engaging parts (9) is formed on the first needle protection part (5.1) and the second needle protection part (5.2), and wherein
the engaging parts (9) has a first engaging part (9.1) formed on the first needle protection part (5.1) and a second engaging part (9.2) formed on the second needle protection part (5.2).

8. The needle cover (1, 1', 1") according to any one of claims 1 to 7, wherein
a handle (6) is formed at a terminal (DE) side end of the needle introduction part (3), and wherein the handle (6) is formed to erect from a horizontal axis (HL) toward the top (U) side and formed in an approximately plate shape or an approximately band shape.

9. The needle cover (1', 1") according to any one of claims 1 to 4 and 6 to 8, wherein
the needle introduction part (3) has a bottom wall (3WU') formed to incline upward in the terminal (DE) direction relative to the horizontal axis (HL) to have an inclination angle (θ).

10. A winged needle with a needle cover comprising: a winged needle (30); and the needle cover (1, 1', 1") according to any one of claims 1 to 9.
